(19) Europäisches Patentamt
European Patent Office
Office européen des brevets

(11) Veröffentlichungsnummer: **0 213 569 B1**

# (12) EUROPÄISCHE PATENTSCHRIFT

(45) Veröffentlichungstag der Patentschrift: **27.03.91**

(51) Int. Cl.5: **G03C 1/815**, C07C 255/31, C07C 229/32, C07C 317/10

(21) Anmeldenummer: **86111622.6**

(22) Anmeldetag: **22.08.86**

## (54) Fotografisches Aufzeichnungsmaterial mit einem UV-Absorber und neue UV-Absorber.

(30) Priorität: **03.09.85 DE 3531383**

(43) Veröffentlichungstag der Anmeldung:
**11.03.87 Patentblatt 87/11**

(45) Bekanntmachung des Hinweises auf die Patenterteilung:
**27.03.91 Patentblatt 91/13**

(84) Benannte Vertragsstaaten:
**BE DE FR GB NL**

(56) Entgegenhaltungen:
**DE-A- 2 152 191**

(73) Patentinhaber: **Agfa-Gevaert AG**
**W-5090 Leverkusen 1(DE)**

(72) Erfinder: **Öhlschläger, Hans, Dr.**
**Am Katterbach 34**
**W-5060 Bergisch Gladbach 2(DE)**
Erfinder: **Langen, Hans, Dr.**
**Weidengarten 16**
**W-5300 Bonn 1(DE)**
Erfinder: **Sobel, Johannes, Dr.**
**Willi-Baumeister-Strasse 9**
**W-5090 Leverkusen 1(DE)**

**EP 0 213 569 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein fotografisches Aufzeichnungsmaterial mit wenigstens einem UV-Absorber und neue UV-Absorber.

Es ist bekannt, UV-Absorber in fotografischen Aufzeichnungsmaterialien, insbesondere in Silberhalogenidmaterialien einzubringen. Durch UV-Absorber wird der unerwünschte Einfluß von UV-Licht bei der Belichtung oder durch elektrostatische Entladungen vermieden. UV-Absorber können z.B. in gelöster Form oder in einem Ölformer, beispielsweise Trikresylphosphat, in das Aufzeichnungsmaterial eingebracht werden. Hierbei treten aber zum Teil erhebliche Nachteile auf. Beispielsweise sind aus der DE-A-2 541 267 und der US-A-40 45 229 Aminoallylidenmalodinitrile als UV-Absorber für fotografische Aufzeichnungsmaterialien bekannt. Sie neigen aber leicht zur Aggregatbildung, was eine unerwünscht breite Absorptionsbande mit niedriger Absorption zur Folge hat. Die bis in den blauempfindlichen Spektralbereich reichenden Flanken der Absorptionsbande verursachen daher eine gelbe Anfärbung des fertigen Bildes und eine verringerte Blauempfindlichkeit des Aufzeichnungsmaterials. Man hat versucht, dieses Problem dadurch zu lösen, daß der UV-Absorber in hochverteilter Form in einen sogenannten beladbaren Latex eingebracht wird, siehe DE-A 2 541 230 und 2 541 274. Die Verwendung polymerer UV-Absorber ist z.B. bekannt aus DE-A 3 313 574, 3 327 464, US 4 307 184 und DE-A 3 401 455. Auch diese Form der Einbringung befriedigt noch nicht alle Ansprüche. Im allgemeinen ist eine außerordentlich hohe Emulgatormenge erforderlich, so daß eine sehr hohe Schichtbelastung mit Emulgatoren auftritt. Hohe Emulgatormengen haben neben der hohen Schichtbelastung weitere ungünstige Eigenschaften, z.B. unbefriedigende Schärfe, Schäumen bei der Verarbeitung, Verschlechterung der Bruchfestigkeit und Ausschwitzen bei der Lagerung.

Der Erfindung liegt die Aufgabe zugrunde, ein verbessertes fotografisches Aufzeichnungsmaterial mit einem UV-Absorber und verbesserte UV-Absorber aufzufinden.

Es wurde nun ein fotografisches Aufzeichnungsmaterial mit einem UV-Absorber gefunden, wobei der UV-Absorber folgende Gruppierung 1) aufweist:

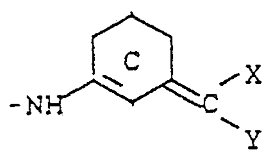

1)

und wobei der Cyclohexenring C substituiert sein kann und X und Y gleich oder verschieden sind und einen elektronenziehenden Rest bedeuten.

In einer bevorzugten Ausführungsform ist die Gruppierung 1) Teil einer hochmolekularen Verbindung.

In einer anderen bevorzugten Ausführungsform weist der UV-Absorber folgende Formel 2) auf

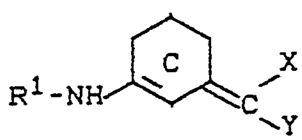

2)

worin der Cyclohexenring C substituiert sein kann mit auf dem Gebiet der UV-Absorber üblichen Substituenten und worin

$R^1$ ein gesättigter oder ungesättigter aliphatischer Kohlenwasserstoffrest, der seinerseits substituiert sein kann mit bei UV-Absorbern üblichen Substituenten, ist

X,Y gleich oder verschieden sind und einen elektronenziehenden Rest bedeuten.

In einer besonders bevorzugten Ausführungsform bedeuten in der Formel 2):

$R^1$ einen gegebenenfalls substituierten gesättigten oder ungesättigten aliphatischen Kohlenwasserstoffrest mit 1 bis 24 C-Atomen, der seinerseits substituiert sein kann, insbesondere mit Halogen wie Chlor oder Brom, Aryl wie Phenyl oder substituiertes Phenyl, Hydroxy, Alkoxy wie Methoxy, Butoxy, Hexadecyloxy, Aryloxy wie Phenoxy, Bistert.-butylphenoxy oder $COOR^5$,

X,Y: gleich oder verschieden: CN, $COOR^4$, $CONHR^4$, $COR^4$, $SO_2R^4$,

$R^4$, $R^5$; gleich oder verschieden: einen Alkylrest mit 1 bis 18 C-Atomen oder Aralkyl mit 7 bis 15 C-Atomen, oder einen Arylrest, wobei diese Reste substituiert oder unsubstituiert sind.

In einer besonders bevorzugten Ausführungsform bedeuten X oder X und Y eine CN-Gruppe.

Ganz besonders bevorzugte UV-Absorber entsprechen folgender Formel 3)

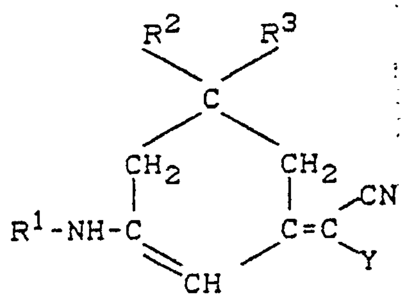

3)

worin

R[1] und Y die oben angegebenen Bedeutungen, insbesondere die bevorzugten Bedeutungen haben und worin

$R^2$,$R^3$ gleich oder verschieden sind und Wasserstoff oder einen gegebenenfalls substituierten Alkylrest mit 1 bis 4 C-Atomen bedeuten.

Besonders bevorzugte Verbindungen der Formel 2) sind in folgender Tabelle 1 angegeben:

| Nr. | Formel | |
|---|---|---|
| 1.1 | 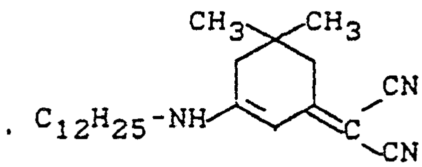 | Fp. 86° C |
| 1.2 | 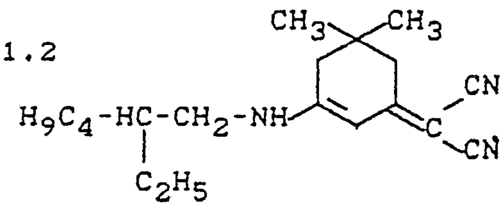 | Fp. 91-95° C |
| 1.3 | 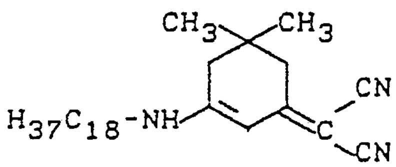 | Fp. 88-90° C |
| 1.4 | 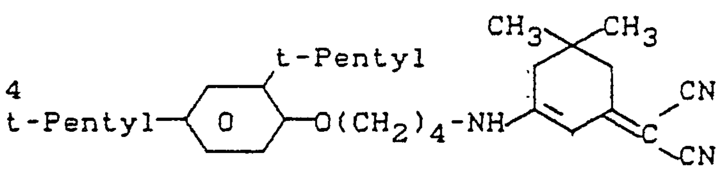 | Fp. 133-134 |
| 1.5 | 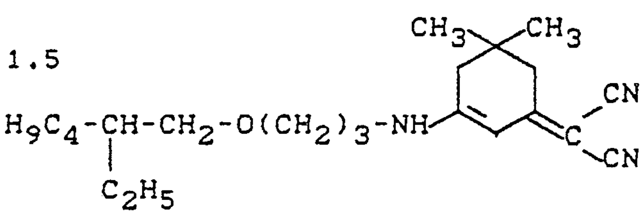 | Öl |
| 1.6 | 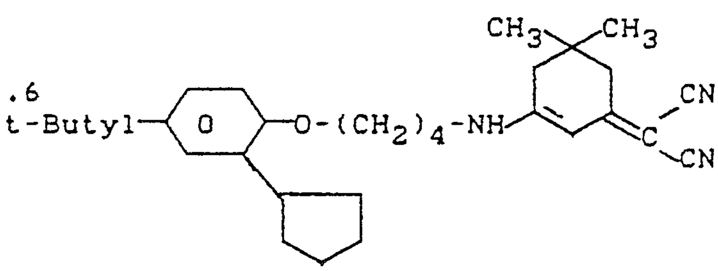 | Fp. 138-139° C |

| Nr. | Formel | |
|---|---|---|
| 1.7 | 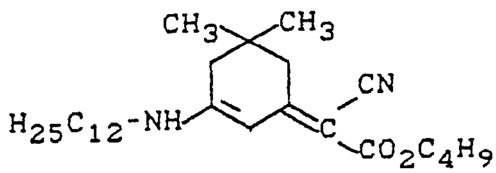 | Fp. 62-65° C |
| 1.8 | 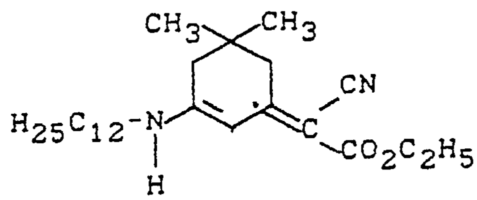 | Fp. 92-94° C |
| 1.9 | 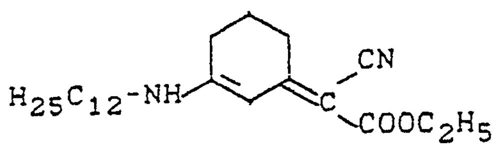 | Fp. 92-94° C |
| 1.10 | 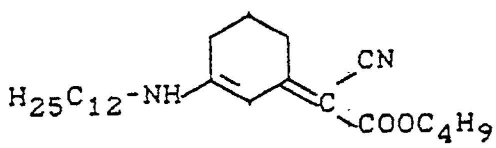 | Fp. 61° C |
| 1.11 | 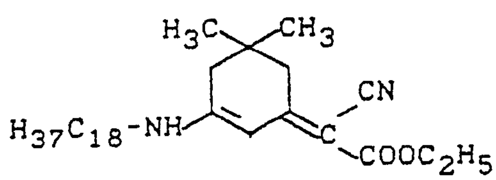 | Fp. 75-77° C |
| 1.12 | 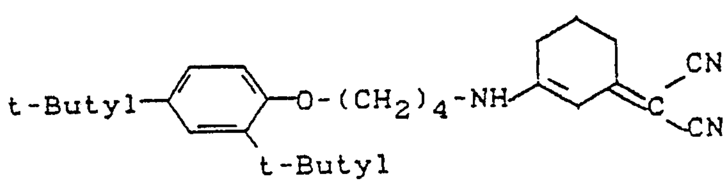 | Fp.165-167° C |

| Nr. | Formel |
|---|---|
| 1.13 | 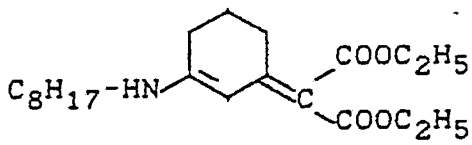 |
| 1.14 | 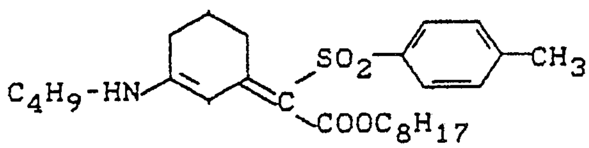 |
| 1.15 | 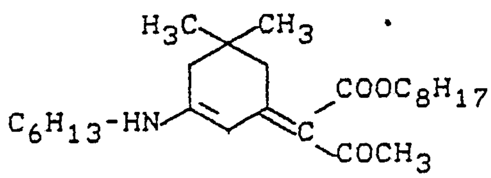 |
| 1.16 | 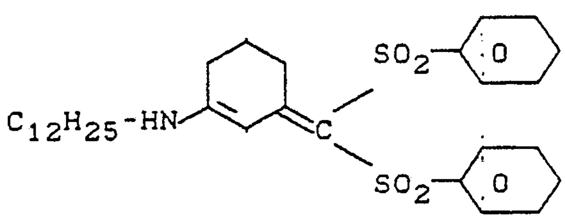 |
| 1.17 | 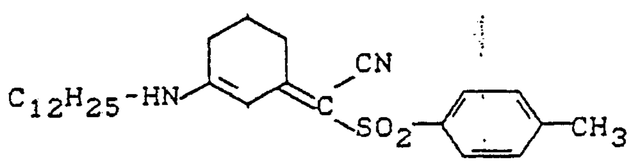 |
| 1.18 | 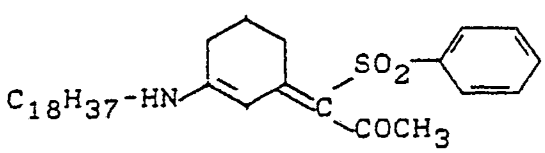 |
| 1.19 | 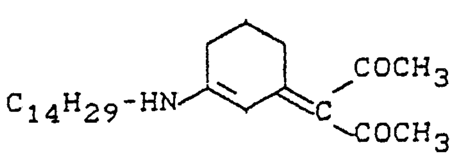 |

| Nr. | Formel |
|---|---|
| 1.20 | 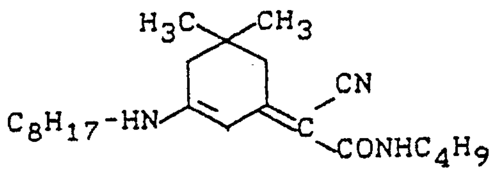 |
| 1.21 | 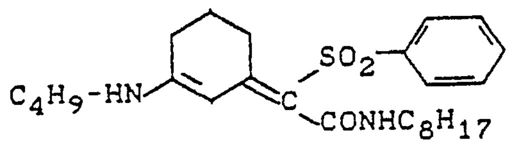 |

Die unter Formel 1) angegebene UV-absorbierende Verbindung kann auch Bestandteil einer hochmolekularen Verbindung HM sein. In diesem Falle kann sie als Gruppierung Q folgender Struktur 4) vorliegen:

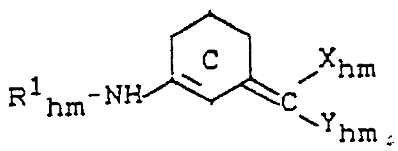 4)

worin

$R^1_{hm}$ die unter $R^1$ angegebene Bedeutung hat und

$X_{hm}$, $Y_{hm}$ gleich oder verschieden sind und die unter X und Y angegebene Bedeutung haben, worin aber wenigstens einer der Substituenten $R^1_{hm}$, $X_{hm}$, $Y_{hm}$ eine chemische Bindung oder ein zweiwertiges Bindeglied bedeutet und darüber die Bindung an die hochmolekulare Verbindung HM vorliegt.

In einer bevorzugten Ausführungsform ist die Gruppierung Q Teil eines Polymeren der folgenden Formel 5)

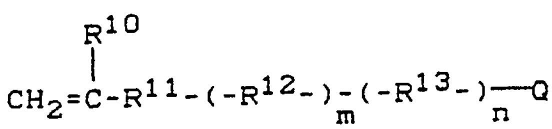 5)

worin

$R^{10}$ ein Wasserstoffatom, eine niedrige Alkylgruppe mit 1 bis 4 C-Atomen oder ein Chloratom ist,

$R^{11}$ eine -CONH-, -COO- oder eine Phenylengruppe ist,

$R^{12}$ eine Alkylengruppe mit 1 bis 20 C-Atomen oder Arylengruppe mit 6 bis 20 C-Atomen,

$R^{13}$ eine -COO-, -OCO-, -CONH-, -NH-CO-O-, -NHCO-, $-SO_2NH-$, $-NHSO_2-$, $-SO_2$-Gruppe oder -O- ist,

m = O oder eine ganze Zahl ist,

n = O oder eine ganze Zahl ist

und worin

Q die unter Formel 4) angegebene Bedeutung hat.

Spezielle Verbindungen der Formel 5) sind beispielhaft in folgender Tabelle 2 angegeben:

| Nr. | Formel |
|---|---|
| 2.1 | 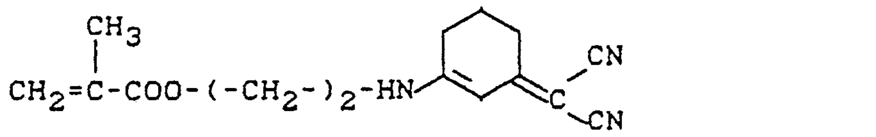 |
| 2.2 | 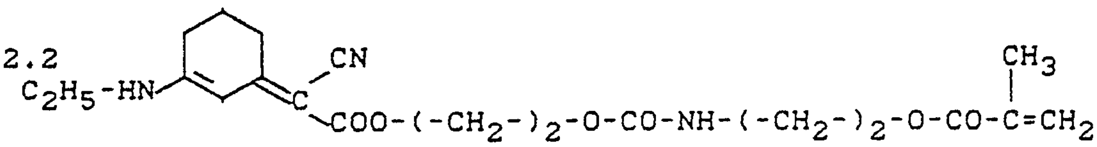 |
| 2.3 | 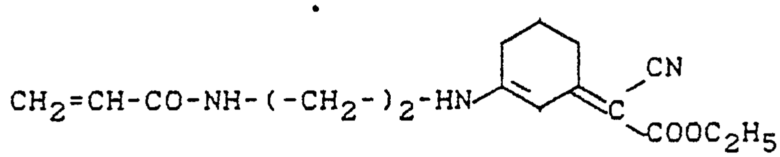 |
| 2.4 | 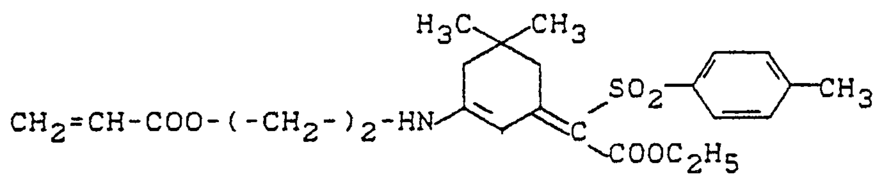 |
| 2.5 | 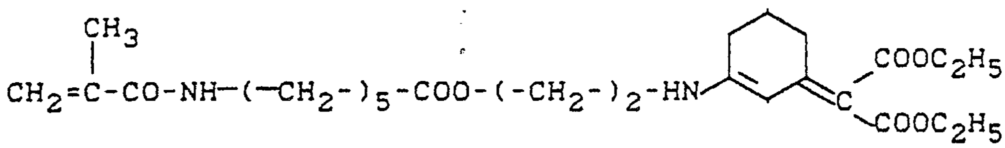 |
| 2.6 | 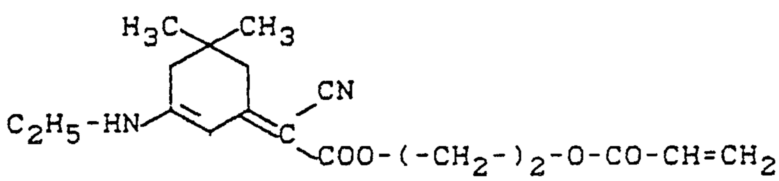 |
| 2.7 | 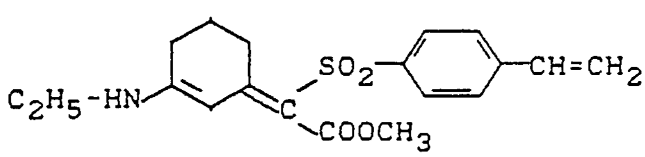 |

| Nr. | Formel |
|---|---|
| 2.8 | 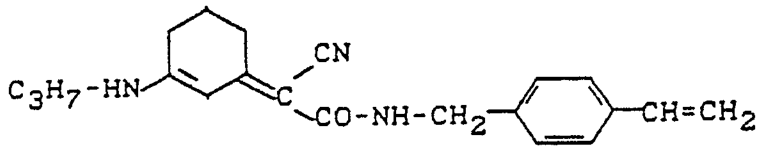 |
| 2.9 | 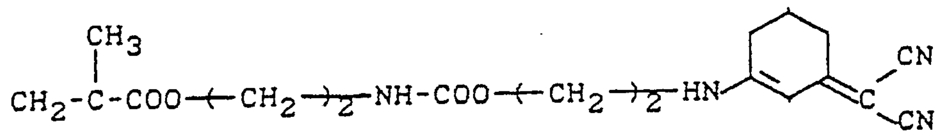 |

Verbindungen der Formel 5) können in an sich bekannter Weise hergestellt und polymerisiert werden, gegebenenfalls unter Copolymerisation üblicher Comonomeren, wobei insbesondere Acrylsäureester, Methacrylsäureester und die aromatischen Vinylverbindungen bevorzugt sind. Es können auch zwei oder mehrere Comonomerverbindungen zusammen verwendet werden, z.B. n-Butylacrylat und Divinylbenzol, Styrol und Methylmethacrylat oder Methacrylat und Methacrylsäure.

Die hochmolekulare Verbindung HM kann auch ein Polyadditions-oder Polykondensationsprodukt sein, welches Urethan-oder Esterverknüpfungen aufweist, mit einer sich wiederholenden Gruppierung G, die sich von der UV-absorbierenden Struktur der allgemeinen Formel 1) ableitet.

In einer besonders bevorzugten Ausführungsform weist die Gruppierung G folgende Struktur 6) auf:

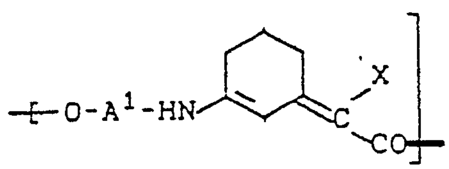 6)

worin

A¹ ein Bindeglied, insbesondere eine Alkylengruppe mit 1 bis 20 Kohlenstoffatomen (beispielsweise eine Methylengruppe, eine Ethylengruppe, eine Trimethylengruppe, eine 2-Hydroxytrimethylengruppe, eine Pentamethylengruppe, eine Hexamethylengruppe, eine Ethylengruppe, eine Propylengruppe oder eine Arylen- oder Cycloalkylengruppe bedeutet und

X ein elektronenziehender Rest ist, insbesondere CN, $COOR^4$, $CONHR^4$, $COR^4$, $SO_2R^4$, wobei $R^4$ die oben angegebene Bedeutung hat,
und wobei die Gruppierung G über das Sauerstoffatom und/ oder die CO-Gruppe mit dem Rest von der Verbindung HM verknüpft ist.

Besonders bevorzugte spezielle Verbindungen sind in folgender Tabelle 3 angegeben:

| Nr. | Formel |
|---|---|
| 3.1 | 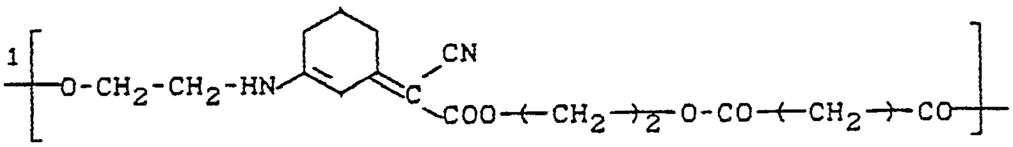 |
| 3.2 | 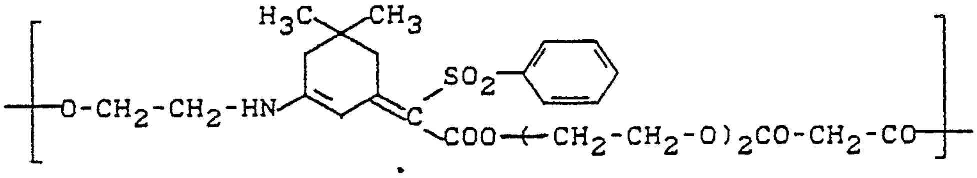 |
| 3.3 | 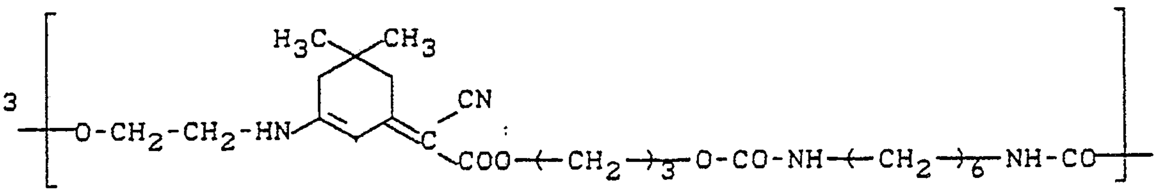 |
| 3.4 | 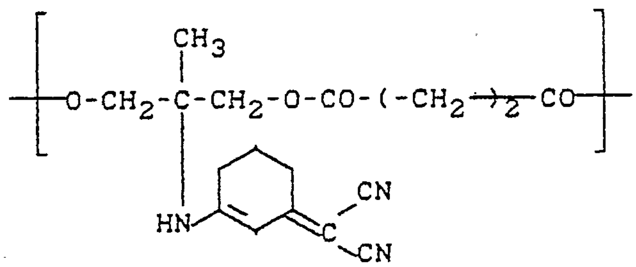 |
| 3.5 | 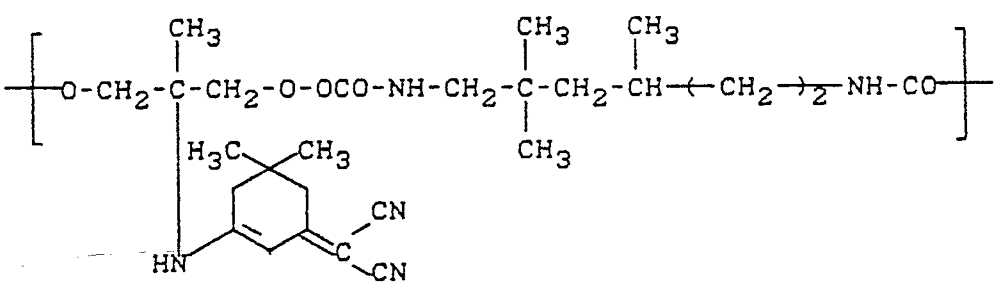 |

| Nr. | Formel |
|---|---|
| 3.6 | 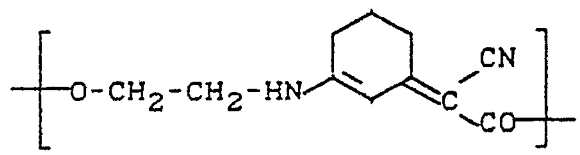 |
| 3.7 | 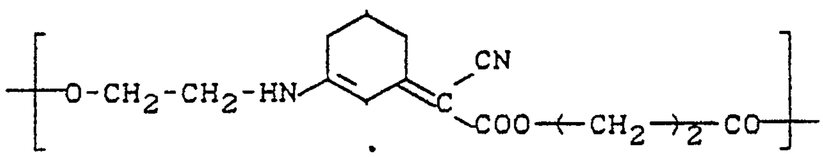 |

Die Molekulargewichte der erfindungsgemäßen Verbindungen 6) sind vorzugsweise im Bereich von 500 bis 5000. Verbindungen mit höheren Molekulargewichten sind ebenfalls geeignet, aber schwieriger herstellbar. Im allgemeinen sind Molekulargewichte im genannten Bereich ausreichend, um eine ausreichende Diffusionsfähigkeit der Polyadditions-bzw. Kondensationsprodukte zu erreichen.

Die Herstellung der erfindungsgemaßen Polyester gemäß Formel 6) kann nach den bekannten Methoden der Polyestersynthese wie z.B. in Ullmanns Encyklopädie der technischen Chemie, 4. Aufl. Bd. 19, S.61 ff., Verlag Chemie, Weinheim, erfolgen. Die Veresterung erfolgt ausgehend von UV-absorbierenden Diolen und Dicarbonsäuren, aktivierten Dicarbonsäuren oder Dicarbonsäurederivaten z.B. Ester, Säurechloride, Säureanhydride, Umsetzungsprodukte aus Carbodiimiden und Dicarbonsäuren, Umsetzungsprodukte aus Dicarbonsäuren und 2-Chlor-2-methylpyridiniumjodid (Macromol. Chem. 185 , 2347 (1984), Dicarbonsäureimidazolide.

Die Synthese der Polyurethane gemäß Formel 6) kann durch Polyaddition von UV-Absorberdiolen mit Diisocyanaten erfolgen gemäß dem in Ullmanns Encyklopädie der technischen Chemie, 4. Aufl., Bd. 19, S.301ff. angegebenen Verfahren. Es ist auch möglich, die beiden Verfahren der Polyester- und Polyurethansynthese zu kombinieren.

Die erfindungsgemäßen Verbindungen lassen sich ausgehend von 1-Aminocyclohexenon-3-Verbindungen der Formel

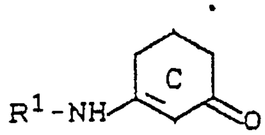

herstellen, worin die Substituenten die bereits angegebene Bedeutung haben. Derartige Verbindungen lassen sich leicht durch Kondensation von Dihydroxyresorcinen mit Aminen darstellen und liefern nach Quaternierung und Umsetzung mit Malonsäurenitril oder anderen geeigneten methylenaktiven Verbindungen die erfindungsgemäßen Verbindungen in guter Ausbeute. Beispielhaft wird im folgenden die Herstellung der Verbindung 1.2 beschrieben, die übrigen Verbindungen können analog erhalten werden:

1-(2-Ethylhexylamino)-5,5-dimethyl-cyclohexenon-3

Eine Mischung von 65 g Ethylhexylamin und 70 g Dimedon in 500 ml Toluol wird 2 Std. bei Raumtemperatur gerührt und danach unter Verwendung eines Wasserabscheiders 3 Std. erhitzt. Dabei werden 8 g Wasser abgetrennt. Das Toluol wird im Vakuum abgedampft, das zurückbleibende Öl erstarrt beim Erkalten zu einem Kristallbrei und kann ohne weitere Reinigung zu weiteren Umsetzungen verwendet werden, Ausbeute praktisch quantitativ. Umkristallisiert aus Cyclohexan: Fp. 68-70 °C.

Verbindung 1.2

125 g 1-(2-Ethylhexylamino)-5,5-dimethyl-cyclohexenon-3 werden mit 53 ml Dimethylsulfat langsam im Ölbad bis 100 °C erhitzt und nach Abklingen der Reaktionstemperatur 40 Min. bei dieser Temperatur weiter gerührt. Die Reaktionsmischung wird auf 60 °C abgekühlt, 85 ml Isopropanol, 33 g Malonitril und 75 ml Triethylamin zugesetzt und 40 Min. im Ölbad von 110 °C erhitzt. Das Gemisch wird abgekühlt, die Verbindung mit Wasser gefällt, abgesaugt und aus Acetonitril umkristallisiert. Die Ausbeute beträgt 127 g = 79 %, Fp. 91-95 °C.

Zum Einsatz in fotografischen Materialien können die erfindungsgemäßen Verbindungen in an sich bekannter Weise dispergiert werden. Dabei kann nach Dispergierung in einer wäßrigen Gelatinelösung das organische Lösungsmittel, das zum Auflösen verwendet wird, aus der Dispersion wieder entfernt werden. Als Lösungsmittel kommen solche in Frage, die ein gewisses Ausmaß an Wasserlöslichkeit aufweisen, so daß sie geeignet sind zur Entfernung durch Wässerung der genudelten Dispersion und solche, die durch Sprühtrocknen, Vakuum- oder Dampfspülen, entfernt werden können. Beispiele für die organischen Lösungsmittel, die zur Entfernung geeignet sind, sind z.B. Ester (beispielsweise Niedrigalkylester, usw.), Niedrigalkylether, Ketone, halogenierte Kohlenwasserstoffe (beispielsweise Methylenchlorid, Trichlorethylen, usw.), fluorierte Kohlenwasserstoffe, Alkohole (beispielsweise Methylalkohol bis Butylalkohol) und Kombinationen davon.

Es kann jeglicher Typ Dispergiermittel bei der Dispergierung des UV-Absorbers verwendet werden. Ionische oberflächenaktive Mittel und insbesondere anionische oberflächenaktive Mittel sind jedoch bevorzugt. Der bevorzugte Mengenbereich von Dispergiermittel liegt zwischen 2% und 15% bezogen auf die eingesetzte Verbindung.

Darüber hinaus ist es möglich, ampholytische oberflächenaktive Mittel zu verwenden, wie C-Cetylbetain, N-Alkylaminopropionate oder N-Alkyliminodipropionate, usw.

Um die Dispersionsstabilität zu verbessern und die Flexibilität der vergossenen Emulsion zu verbessern, kann eine geringe Menge permanenten Lösungsmittels, nämlich eines mit Wasser nicht mischbaren organischen Lösungsmittels mit hohem Siedepunkt (d.h. über 200 °C) beispielsweise Dibutylphosphat und/oder Trikresylphosphat usw. zugesetzt werden. Es ist nötig, daß die Konzentration des permanenten Lösungsmittels ausreichend gering ist, um das Polymere zu plastifizieren, während es in einem Zustand eines festen Teilchens gehalten wird. Darüber hinaus ist es bei Verwendung eines permanenten Lösungsmittels wichtig, daß dessen Menge so gering wie möglich ist, so daß die Dicke der endgültigen Emulsionsschicht oder der hydrophilen Kolloidschicht verringert wird, um eine gute Schärfe aufrechtzuerhalten.

Die erfindungsgemäßen Verbindungen können aber auch auf einen Latex aufgezogen werden.

Bei den fotografischen Aufzeichnungsmaterialien handelt es sich bevorzugt um fotografische Aufzeichnungsmaterialien mit wenigstens einer lichtempfindlichen Silberhalogenidemulsionsschicht und gegebenenfalls weiteren Hilfsschichten.

Die erfindungsgemäßen Verbindungen können z.B. in eine Bindemittelschicht oder in eine lichtempfindliche Silberhalogenidemulsionsschicht eingebracht werden. Die Silberhalogenidemulsionsschichten können in Teilschichten gleicher spektraler Empfindlichkeit aufgeteilt werden, die unterschiedlich empfindlich sind. Die erfindungsgemäß zu verwendenden Verbindungen können z.B. in eine hochempfindliche blauempfindliche Teilschicht eingebracht werden. In einer anderen bevorzugten Ausführungsform werden sie in eine oben liegende UV-Filterschicht eingebracht, die oberhalb aller lichtempfindlichen Schichten angeordnet ist.

Die zu verwendende Menge hängt von dem Verwendungszweck ab und kann in üblicher Weise leicht optimiert werden. Bevorzugte Mengen sind z.B.: 50 - 1000 mg/$m^2$.

Außer den bereits genannten Schichten können weitere, nicht lichtempfindliche Hilfsschichten in dem erfindungsgemäßen farbfotografischen Aufzeichnungsmaterial vorhanden sein, z.B. Haftschichten, Lichthofschutzschichten oder Deckschichten, insbesondere Zwischen schichten zwischen den lichtempfindlichen Schichten, wodurch die Diffusion von Entwickleroxidationsprodukten aus einer Schicht in eine andere wirksam unterbunden werden soll.

Den lichtempfindlichen Silberhalogenidemulsionsschichten sind vorzugsweise Farbkuppler zugeordnet, die mit Farbentwickleroxidationsprodukten unter Bildung eines nicht-diffundierenden Farbstoffes reagieren. Zweckmäßigerweise sind die Farbkuppler nicht diffundierend in der lichtempfindlichen Schicht selbst oder in enger Nachbarschaft hierzu untergebracht. Es kann aber auch zweckmäßig sein, wenigstens einige Kuppler mit begrenzter Diffusionsfähigkeit zu verwenden.

So kann die rotempfindliche Schicht beispielsweise einen nicht diffundierenden Farbkuppler zur Erzeugung des blaugrünen Teilfarbenbildes enthalten, in der Regel einen Kuppler vom Phenol- oder α-Naphtholtyp. Die grünempfindliche Schicht kann beispielsweise mindestens einen nicht diffundierenden Farbkuppler zur Erzeugung des purpurnen Teilfarbenbildes enthalten, wobei üblicherweise Farbkuppler vom

Typ des 5-Pyrazolons verwendet werden. Die blauempfindliche Schicht kann beispielsweise mindestens einen nicht-diffundierenden Farbkuppler zur Erzeugung des gelben Teilfarbenbildes, in der Regel einen Farbkuppler mit einer offenkettigen Ketomethylengruppierung enthalten. Bei den Farbkupplern kann es sich z.B. um 6-, 4- und um 2-Äquivalentkuppler handeln. Geeignete Kuppler sind beispielsweise bekannt aus den Veröffentlichungen "Farbkuppler" von W. Pelz in "Mitteilungen aus den Forschungslaboratorien der Agfa, Leverkusen/München, Band III, Seite 111 (1961), K. Venkataraman in "The Chemistry of Synthetic Dyes", Vol. 4, 341 bis 387, Academic Press (1971) und T.H. James, "The Theory of the Photographic Process", 4. Ed., S. 353-362, sowie aus der Zeitschrift Research Disclosure Nr. 17643 vom Dezember 1978, Abschnitt VII, veröffentlicht von Industrial Opportunities Ltd., Homewell Havant, Hampshire, PO9 1 EF in Großbritannien. Das Aufzeichnungsmaterial kann weiterhin DIR-Verbindungen und Weißkuppler, die bei Reaktion mit Farbentwickleroxidationsprodukten keinen Farbstoff ergeben, enthalten. Die von den DIR-Verbindungen abspaltbaren Inhibitoren können unmittelbar oder über nicht hemmende Zwischenverbindungen abgespalten werden. Verwiesen wird auf GB 953 454, US 3 632 345, US 4 248 962 und GB 2 072 363.

Die verwendeten lichtempfindlichen Silberhalogenidemulsionen können als Halogenid Chlorid, Bromid und Iodid bzw. Mischungen davon enthalten. In einer bevorzugten Ausführungsform besteht der Halogenidanteil wenigstens einer Schicht zu 0 bis 12 Mol-% aus AgI, zu 0 bis 50 Mol-% aus AgCl und zu 50 bis 100 % aus AgBr. In einer bevorzugten Ausführungsform handelt es sich um überwiegend kompakte Kristalle, die z.B. kubisch oder oktaedrisch sind oder Übergangsformen aufweisen. Sie lassen sich dadurch kennzeichnen, daß sie im wesentlichen eine Dicke von mehr als 0,2 σm aufweisen. Das durchschnittliche Verhältnis von Durchmesser zu Dicke ist bevorzugt kleiner als 8:1, wobei gilt, daß der Durchmesser eines Kornes definiert ist als der Durchmesser eines Kreises mit einem Kreisinhalt entsprechend der projizierten Fläche des Kornes. In einer anderen bevorzugten Ausführungsform können alle oder einzelne Emulsionen aber auch im wesentlichen tafelförmige Silberhalogenidkristalle aufweisen, bei denen das Verhältnis von Durchmesser zu Dicke größer als 8:1 ist.

Die Emulsionen können chemisch sensibilisiert sein. Zur chemischen Sensibilisierung der Silberhalogenidkörner sind die üblichen Sensibilisierungsmittel geeignet. Besonders bevorzugt sind schwefelhaltige Verbindungen, beispielsweise Allylisothiocyanat, Allylthioharnstoff und Thiosulfate. Geeignet als chemische Sensibilisatoren sind auch Edelmetalle bzw. Edelmetallverbindungen wie Gold, Platin, Palladium, Iridium, Ruthenium oder Rhodium. Diese Methode der chemischen Sensibilisierung ist in dem Artikel von R. Koslowsky, Z.Wiss.Phot. 46 , 65-72 (1951), beschrieben. Es ist ferner möglich, die Emulsionen mit Polyalkylenoxid-Derivaten zu sensibilisieren. Verwiesen wird weiter auf die oben angegebene Research Disclosure Nr. 17 643, Abschnitt III.

Die Emulsionen können in an sich bekannter Weise optisch sensibilisiert werden, z.B. mit den üblichen Polymethinfarbstoffen, wie Neutrocyaninen, basischen oder sauren Carbocyaninen, Rhodacyaninen, Hemicyaninen, Styrylfarbstoffen, Oxonolen und ähnlichen. Derartige Sensibilisatoren sind von F.M. Hamer in "The Cyanine Dyes and related Compounds", (1964), beschrieben. Verwiesen sei diesbezüglich insbesondere auf Ullmanns Enzyklopädie der technischen Chemie, 4. Auflage, Band 18, Seiten 431 ff und auf die oben angegebene Research Disclosure Nr. 17 643, Abschnitt IV.

Zusätzlich zu den erfindungsgemäß zu verwendenden Verbindungen können die üblichen Antischleiermittel und Stabilisatoren verwendet werden. Als Stabilisatoren sind besonders geeignet Azaindene, vorzugsweise Tetra- oder Pentaazaindene, insbesondere solche, die mit Hydroxyl- oder Aminogruppen substituiert sind. Derartige Verbindungen sind z.B. in dem Artikel von Birr, Z.Wiss.Phot. 47 , 1952), S. 2-58, beschrieben. Weitere geeignete Stabilisatoren und Antischleiermittel sind in der oben angegebenen Research Disclosure Nr. 17 643 in Abschnitt IV angegeben.

Die Bestandteile des fotografischen Materials können nach üblichen, bekannten Methoden eingearbeitet werden. Wenn es sich um wasser- oder alkalilösliche Verbindungen handelt, können sie in Form von wäßrigen Lösungen, gegebenenfalls unter Zusatz von mit Wasser mischbaren organischen Lösungsmitteln wie Ethanol, Aceton oder Dimethylformamid, zugesetzt werden. Wenn sie wasser- bzw. alkaliunlöslich sind, können sie in an sich bekannter Weise in dispergierter Form in die Aufzeichnungsmaterialien eingearbeitet werden. Zum Beispiel kann man eine Lösung dieser Verbindungen in einem niedrig siedenden organischen Lösungsmittel direkt mit der Silberhalogenidemulsion oder zunächst mit einer wäßrigen Gelatinelösung vermischen und darauf das organische Lösungsmittel entfernen. Die so erhaltene Dispersion der jeweiligen Verbindung kann anschließend mit der Silberhalogenidemulsion vermischt werden. Gegebenenfalls verwendet man zusätzlich noch sogenannte Ölformer, in der Regel höhersiedende organische Verbindungen, die die zu dispergierenden Verbindungen in Form öliger Tröpfchen einschließen. Verwiesen wird in diesem Zusammenhang beispielsweise auf die US-Patentschriften 2 322 027, 2 533 514, 3 689 271, 3 764 336 und 3 765 897. Es ist auch möglich, z.B. Kuppler in Form beladener Latices einzubauen, siehe DE-OS 2 541 274 und EP-A 14 921. Weiterhin können die Bestandteile auch als Polymere im Material festgelegt werden,

siehe z.B. DE-OS 2 044 992, US 3 370 952 und US 4 080 211.

Für die erfindungsgemäßen Materialien können die üblichen Schichtträger verwendet werden, z.B. Träger aus Celluloseestern, z.B. Celluloseacetat und aus Polyestern. Geeignet sind ferner Papierträger, die gegebenenfalls beschichtet sein können z.B. mit Polyolefinen, insbesondere mit Polyethylen oder Polypropylen. Verwiesen wird diesbezüglich auf die oben angegebene Research Disclosure Nr. 17 643, Abschnitt XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Verwiesen wird auf die in der oben angegebenen Research Disclosure 17 643 in Abschnitt IX angegebenen Bindemittel.

Die Schichten des fotografischen Materials können in der üblichen Weise gehärtet sein, beispielsweise mit Härtern des Epoxidtyps, des heterocyclischen Ethylenimins und des Acryloyltyps. Weiterhin ist es auch möglich, die Schichten gemäß dem Verfahren der deutschen Offenlegungsschrift 2 218 009 zu härten, um farbfotografische Materialien zu erzielen, die für eine Hochtemperaturverarbeitung geeignet sind. Es ist ferner möglich, die fotografischen Schichten bzw. die farbfotografischen Mehrschichtenmaterialien mit Härtern der Diazin- Triazin- oder 1,2-Dihydrochinolin-Reihe zu härten oder mit Härtern vom Vinylsulfon-Typ. Weitere geeignete Härtungsmittel sind aus den deutschen Offenlegungsschriften 2 439 551, 2 225 230, 2 317 672 und aus der oben angegebenen Research Disclosure 17 643, Abschnitt XI bekannt.

Weitere geeignete Zusätze werden in der Research Disclosure 17 643 und in "Product Licensing Index" von Dezember 1971, Seiten 107-110, angegeben.

Geeignete Farbentwicklersubstanzen für das erfindungsgemäße Material sind insbesondere solche vom p-Phenylendiamintyp, z.B. 4-Amino-N,N-diethyl-anilinhydrochlorid; 4-Amino-3-methyl-N-ethyl-N-ß-(methansulfonamido)-ethylanilinsulfathydrat; 4-Amino-3-methyl-N-ethyl-N-ß-hydroxyethylanilinsulfat; 4-Amino-N-ethyl-N-(2-methoxyethyl)-m-toluidin-di-p-toluolsulfonsäure und N-Ethyl-N-ß-hydroxyethyl-p-phenylendiamin. Weitere brauchbare Farbentwickler sind beispielsweise beschrieben in J.Amer.Chem.Soc. 73, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren, insbesondere z.B. Ethylendiamintetraessigsäure, Nitrilotriessigsäure, Iminodiessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Die in den folgenden Beispielen angegebenen Vergleichsverbindungen haben folgende Struktur:

Vergleichsverbindung A:

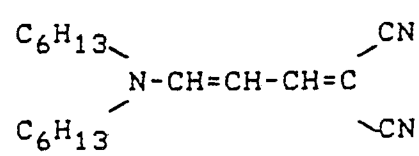

Vergleichsverbindung B:

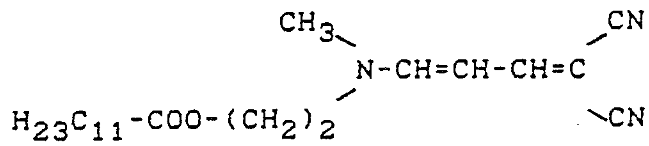

Beispiel 1

Verbindung 1.5 wurde in Gelatine nach der nachfolgend beschriebenen allgemeinen Vorschrift dispergiert. Die erhaltene Dispersion wurde so auf eine transparente Triacetatfolie aufgetragen, daß sich auf einer Fläche von 1 $m_x$ 0,2 mmol des UV-Absorbers und 0,6 g Gelatine befanden. Danach wurde das Absorptionsspektrum ermittelt. Als Referenz wurde eine Schicht die keinen UV-Absorber enthielt, eingesetzt. In gleicher Weise wie Verbindung 1.5 wurden dann noch jeweils 0,2 mmol der Verbindung 1.8 sowie eine Abmischung von 20 % der Verbindung 1.7 mit 80 % der Verbindung 1.2 ebenfalls auf Triacetatfolien aufgetragen. Zum Vergleich kamen jeweils 0,2 mmol der Verbindung A und B.

Allgemeine Dispergiervorschrift:

10 g UV-Absorber wurden zusammen mit 10 g Trikresylphosphat und 30 ml Essigester bei 55° C gelöst und in 150 ml 7,5 %ige Gelatine, die ebenfalls auf 55° C erhitzt war unter Verwendung eines Dispergiermittels dispergiert. Die erhaltene Dispersion wurde anschließend im Vakuum von Essigester befreit und bei 6° C erstarrt.

Wie aus den Spektren der Abb. 1 zu ersehen ist, bildet der neue UV-Absorbertyp im langwelligen UV-Bereich ein deutliches Maximum mit steil abfallender langwelliger Flanke aus, während die Vergleichsverbindungen eine geringere Extinktion mit breiter Absorptionsbande zeigen verbunden mit einer flacher abfallenden Flanke, was in der Praxis zur Empfindlichkeitseinbuße und D-min-Erhöhung führen kann. In Abbildung 1 bedeuten:

- Kurve 5: Material mit UV-Absorber 1.5
- Kurve 8: Material mit UV-Absorber 1.8
- Kurve 7/2: Material mit der Abmischung aus den UV-Absorbern 1.7 und 1.2
- Kurve A: Material mit dem Vergleichs-UV-Absorber A
- Kurve B: Material mit dem Vergleichs-UV-Absorber B.

Beispiel 2

Es wurden folgende hochempfindliche Color-Negativ-Materialien hergestellt durch Auftragen der im folgenden angegebenen Schichten auf einen üblichen Schichtträger:

- 1. Schicht: Antihaloschicht mit kolloidalem Silber, Gelatine und einem Octylhydrochinon
- 2. Schicht: Zwischenschicht
- 3. Schicht: rotempfindliche, niedrigempfindliche Silberbromidiodidemulsionsschicht mit einem üblichen Cyan-Kuppler, einem DIR-Kuppler und einem Rotmaskenkuppler
- 4. Schicht: rotempfindliche, hochempfindliche Silberbromidiodidemulsionsschicht (10 Mol-% Iodid) und dem in der 3. Schicht angegebenen Cyankuppler
- 5. Schicht: Zwischenschicht mit Gelatine und einem Octylhydrochinon
- 6. Schicht: grünempfindliche, niedrigempfindliche Silberbromidiodidschicht (5 Mol-% Silberbromid) mit einem Purpurkuppler, einem Gelb-Maskenkuppler und einem DIR-Kuppler
- 7. Schicht: grünempfindliche, hochempfindliche Silberbromidiodidschicht (9 Mol-% Iodid) mit den in Schicht 6 angegebenen Purpur- und Maskenkupplern
- 8. Schicht: Zwischenschicht mit Gelatine und einem Octylhydrochinon
- 9. Schicht: Filtergelbschicht mit kolloidalem Silber
- 10. Schicht: blauempfindliche, niedrigempfindliche Silberbromidiodidemulsionsschicht (6 Mol-% Iodid) und 1,05 g pro $m^2$ eines üblichen Gelbkupplers. Silberauftrag: entsprechend 1,0 g Silbernitrat pro $m^2$.
- 11. Schicht: blauempfindliche, hochempfindliche Silberbromidiodidschicht (8,5 Mol-% Silberiodid) und 0,26 g pro $m^2$ eines üblichen Gelbkupplers. Silberauftrag: entsprechend 0,8 g Silbernitrat pro $m^2$.
- 12. Schicht: UV-Absorberschicht
- 13. Schicht: Schutzschicht mit Gelatine und einem Carbamoylpyridiniumsalz als Härtungsmittel.

Die genannten Schichten enthalten zusätzlich zu den genannten Bestandteilen weitere übliche Bausteine.

In die UV-Absorberschicht 12 wurden jeweils 0,2 mmol der Verbindungen 1.2, 1.4, 1.5, 1.6 und 1.8 sowie die Vergleichsverbindungen A und B nach der in Beispiel 1 beschriebenen Rezeptur eingelagert.

Mit den hergestellten Materialien wurde nun eine gelbe Objektfarbe, deren Remissionsspektrum in Abbildung 2 zu ersehen ist, aufgenommen. Gleichzeitig wurde ein neutral abgestimmter Graukeil mit aufgenommen. Nach Entwicklung in dem bei E. Ch. Gehret, Brit. J. of Photography 1974, S. 567 beschriebenen Prozeß wurde das Negativ auf ein herkömmliches Colorpapier kopiert. Die erhaltenen Prints wurden, um einen Vergleich zu ermöglichen, mit Hilfe des mitaufgenommenen Graukeils exakt grau kopiert. Danach wurden die prints der mitaufgenommenen Objektfarbe mit einem Aufsichtsdensitometer des Typs Macbeth RD 400, das mit einem Wratten-Filter No. 47 ausgestattet war, gemessen. Aus der nachfolgenden Zusammenstellung ist zu ersehen, daß die erfindungsgemäßen UV-Absorber eine höhere gb-Dichte der wiedergegebenen gelben Objektfarbe in der Kopie, d.h. eine bessere Farbsättigung ohne D-min-Erhöhung und ohne Empfindlichkeitsverlust als die Vergleichsverbindungen ergaben.

| Aufgetragener UV-Absorber (Nr.) | | Neg.-Empf.* (log H) | D-min | Dichte/gb in der Kopie |
|---|---|---|---|---|
| ohne | | -3,75 | 0,84 | 0,85 |
| 1.2 | erfindungsgem. | -3,65 | 0,85 | 1,38 |
| 1.4 | " | -3,67 | 0,85 | 1,36 |
| 1.5 | " | -3,66 | 0,84 | 1,32 |
| 1.6 | " | -3,67 | 0,84 | 1,35 |
| 1.8 | " | -3,64 | 0,85 | 1,43 |
| A | Vergleich | -3,62 | 0,87 | 1,25 |
| B | Vergleich | -3,64 | 0,86 | 1,31 |

*) Ein Anwachsen von 0,3 log H korrespondiert mit einem Rückgang auf die halbe Empfindlichkeit.

## Ansprüche

1. Fotografisches Aufzeichnungsmaterial mit wenigstens einem UV-Absorber, dadurch gekennzeichnet, daß der UV-Absorber folgende Gruppierung 1) aufweist

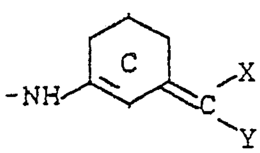

1)

wobei der Cyclohexenring C gegebenenfalls substituiert sein kann und X und Y gleich oder verschieden sind und einen elektronenziehenden Rest bedeuten.

2. Fotografisches Aufzeichnungsmaterial nach Anspruch 1, dadurch gekennzeichnet, daß die Gruppierung 1) Teil einer hochmolekularen Verbindung ist.

3. Fotografisches Aufzeichnungsmaterial nach Anspruch 1, dadurch gekennzeichnet, daß der UV-Absorber folgender Formel 2) entspricht

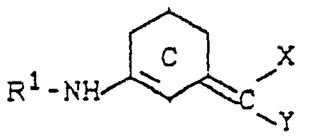

2)

worin der Cyclohexenring C substituiert sein kann und worin bedeuten

- $R^1$ einen gesättigten oder ungesättigten aliphatischen Kohlenwasserstoffrest, der gegebenenfalls substituiert sein kann und worin
- X,Y gleich oder verschieden sind und einen elektronenziehenden Rest bedeuten.

4. Fotografisches Aufzeichnungsmaterial nach Anspruch 3, dadurch gekennzeichnet, daß

- $R^1$ einen gesättigten oder ungesättigten aliphatischen Kohlenwasserstoffrest mit 1 bis 24 C-Atomen bedeutet, der gegebenenfalls seinerseits substituiert sein kann und worin
- X,Y gleich oder verschieden sind und CN, $COOR^4$, $CONHR^4$, $COR^4$ oder $SO_2R^4$
- $R^4$ einen Alkylrest mit 1 bis 18 C-Atomen oder Aralkyl mit 7 bis 15 C-Atomen oder Aryl bedeuten.

5. Fotografisches Aufzeichnungsmaterial nach Anspruch 1, dadurch gekennzeichnet, daß der UV-Absorber folgender Formel 3 entspricht

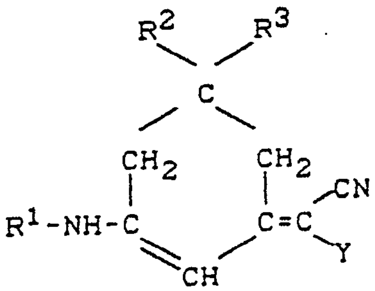

3)

worin bedeuten

- $R^1$ einen gegebenenfalls substituierten gesättigten oder ungesättigten aliphatischen Kohlenwasserstoffrest
- $R^2$,$R^3$ die gleich oder verschieden sind, Wasserstoff oder einen gegebenenfalls substituierten Alkylrest mit 1 bis 4 C-Atomen
- Y CN, $COOR^4$, $CONHR^4$, $COR^4$ oder $SO_2R^4$
- $R^4$ einen Alkylrest mit 1 bis 18 C-Atomen oder Aralkyl mit 7 bis 15 C-Atomen oder Aryl.

6. Fotografisches Aufzeichnungsmaterial nach Anspruch 1, dadurch gekennzeichnet, daß der UV-Absorber in einer lichtempfindlichen Silberhalogenidemulsionsschicht enthalten ist.

7. Fotografisches Aufzeichnungsmaterial nach Anspruch 1, dadurch gekennzeichnet, daß der UV-Absorber in einer lichtunempfindlichen Filterschicht enthalten ist.

8. UV-absorbierende Verbindungen, die folgende Gruppierung aufweisen:

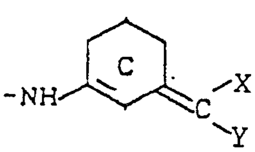

wobei der Cyclohexenring C gegebenenfalls substituiert sein kann und X und Y gleich oder verschieden

sind und einen elektronenziehenden Rest bedeuten.

9. UV-absorbierende Verbindungen nach Anspruch 8, dadurch gekennzeichnet, daß sie folgender Formel entsprechen:

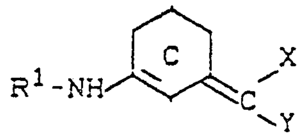

worin der Cyclohexenring C substituiert sein kann und worin bedeuten

$R^1$ einen gesättigten oder ungesättigten aliphatischen Kohlenwasserstoffrest, der gegebenenfalls substituiert sein kann und worin

X,Y gleich oder verschieden sind und einen elektronenziehenden Rest bedeuten.

## Claims

1. A photographic recording material containing at least one UV absorber, characterized in that the UV absorber contains the following group a)

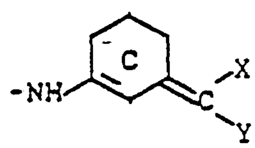

1)

in which the cyclohexene ring C may optionally be substituted and X and Y may be the same or different and represent an electron-attracting group.

2. A photographic recording material as claimed in claim 1, characterized in that the group 1) is part of a high molecular weight compound.

3. A photographic recording material as claimed in claim 1, characterized in that the UV absorber corresponds to the following formula 2)

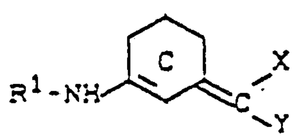

2)

in which the cyclohexene ring C may be substituted and in which

$R^1$ is a saturated or unsaturated aliphatic hydrocarbon radical which may optionally be substituted and

X and Y may be the same or different and represent an electron-attracting group.

4. A photographic recording material as claimed in claim 3, characterized in that

$R^1$ is a saturated or unsaturated aliphatic $C_{1-24}$ hydrocarbon radical which may optionally be substituted and

X and Y may be the same or different and represent CN, $COOR^4$, $CONHR^4$, $COR^4$ and $SO_2R^4$,

$R^4$ is a $C_{1-18}$ alkyl radical or a $C_{7-15}$ aralkyl radical or an aryl radical.

5. A photographic recording material as claimed in claim 1, characterized in that the UV absorber corresponds to formula 3

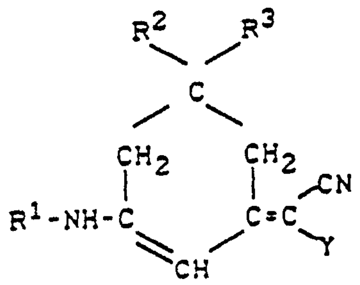

3)

in which

| | |
|---|---|
| $R^1$ | is an optionally substituted saturated or unsaturated aliphatic hydrocarbon radical, |
| $R^2$ and $R^3$ | may be the same or different and represent hydrogen or an optionally substituted $C_{1-4}$ alkyl radical, |
| Y | represents CN, $COOR^4$, $CONHR^4$, $COR^4$ and $SO_2R^4$, |
| $R^4$ | is a $C_{1-18}$ alkyl radical or a $C_{7-15}$ aralkyl radical or an aryl radical. |

6. A photographic recording material as claimed in claim 1, characterized in that the UV absorber is contained in a photosensitive silver halide emulsion layer.

7. A photographic recording material as claimed in claim 1, characterized in that the UV absorber is contained in a non-photosensitive filter layer.

8. UV-absorbing compounds containing the following group:

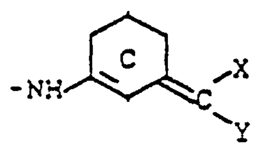

1)

in which the cyclohexene ring C may optionally be substituted and X and Y may be the same or different and represent an electron-attracting group.

9. UV-absorbing compounds as claimed in claim 8, charac terized in that they correspond to the following formula:

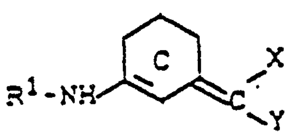

2)

in which the cyclohexene ring C may be substituted and in which

| | |
|---|---|
| $R^1$ | is a saturated or unsaturated aliphatic hydrocarbon radical which may optionally be substituted and |
| X and Y | may be the same or different and represent an electron-attracting group. |

**Revendications**

1. Matériau de reproduction photographique contenant au moins un absorbeur d'UV, caractérisé en ce que l'absorbeur d'UV présente le groupement 1) suivant :

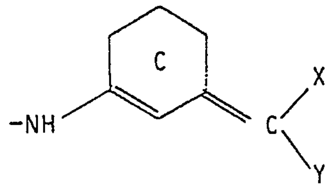

1)

dans lequel le noyau cyclohexène C peut être éventuellement substitué et X et Y sont identiques ou différents et représentent un reste attirant les électrons.

2. Matériau de reproduction photographique selon la revendication 1, caractérisé en ce que le groupement 1) fait partie d'un composé de haut poids moléculaire.

3. Matériau de reproduction photographique selon la revendication 1, caractérisé en ce que l'absorbeur d'UV répond à la formule 2) suivante

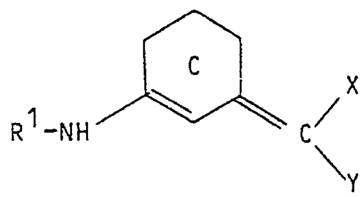

2)

dans laquelle le noyau cyclohexène C peut être substitué et dans laquelle

- $R^1$ représente un reste d'hydrocarbure aliphatique saturé ou insaturé, qui peut éventuellement être substitué et dans laquelle
- X et Y sont identiques ou différents et représentent un reste attirant les électrons.

4. Matériau de reproduction photographique selon la revendication 3, caractérisé en ce que $R^1$ représente un reste d'hydrocarbure aliphatique en $C_1$-$C_{24}$, saturé ou insaturé, qui peut éventuellement être substitué,
X et Y sont identiques ou différents et représentent CN, $COOR^4$, $CONHR^4$, $COR^4$ ou $SO_2R^4$ et
$R^4$ représente un reste alkyle en $C_1$-$C_{18}$, un arylalkyle en $C_7$-$C_{15}$ ou un reste aryle.

5. Matériau de reproduction photographique selon la revendication 1, caractérisé en ce que l'absorbeur d'UV répond à la formule 3) suivante

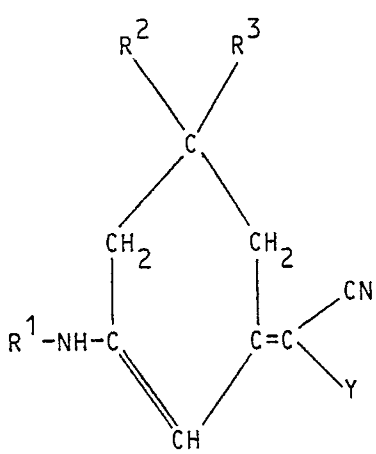

3)

dans laquelle
$R^1$ représente un reste d'hydrocarbure aliphatique saturé ou insaturé, éventuellement substitué,
$R^2$ et $R^3$ sont identiques ou différents et représentent l'hydrogène ou un reste alkyle en $C_1$-$C_4$,

éventuellement substitué,
Y représente CN, $COOR^4$, $CONHR^4$, $COR^4$ ou $SO_2R^4$ et
$R^4$ représente un reste alkyle en $C_1$-$C_{18}$, un reste arylalkyle en $C_7$-$C_{15}$ ou un reste aryle.

6. Matériau de reproduction photographique selon la revendication 1, caractérisé en ce que l'absorbeur d'UV est contenu dans une couche d'émulsion d'halogénure d'argent sensible à la lumière.

7. Matériau de reproduction photographique selon la revendication 1 , caractérisé en ce que l'absorbeur d'UV est contenu dans une couche filtrante insensible à la lumière.

8. Composés absorbant les UV, qui présentent le groupement suivant

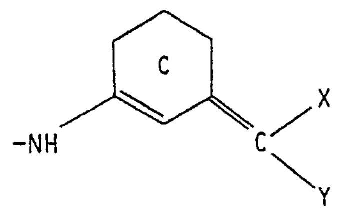

1)

dans laquelle le noyau cyclohexène C peut être substitué et dans laquelle
X et Y sont identiques ou différents et représentent un reste attirant les électrons.

9. Composés absorbant les UV selon la revendication 8, caractérisés en ce qu'ils répondent à la formule 2) suivante

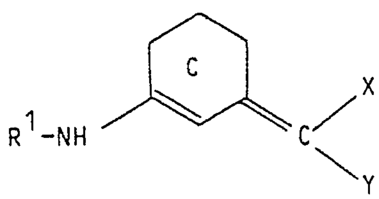

2)

dans laquelle le noyau cyclohexène C peut être substitué et dans laquelle
$R^1$ représente un reste d'hydrocarbure aliphatique saturé ou insaturé, qui peut éventuellement être substitué et dans laquelle
X et Y sont identiques ou différents et représentent un reste attirant les électrons.

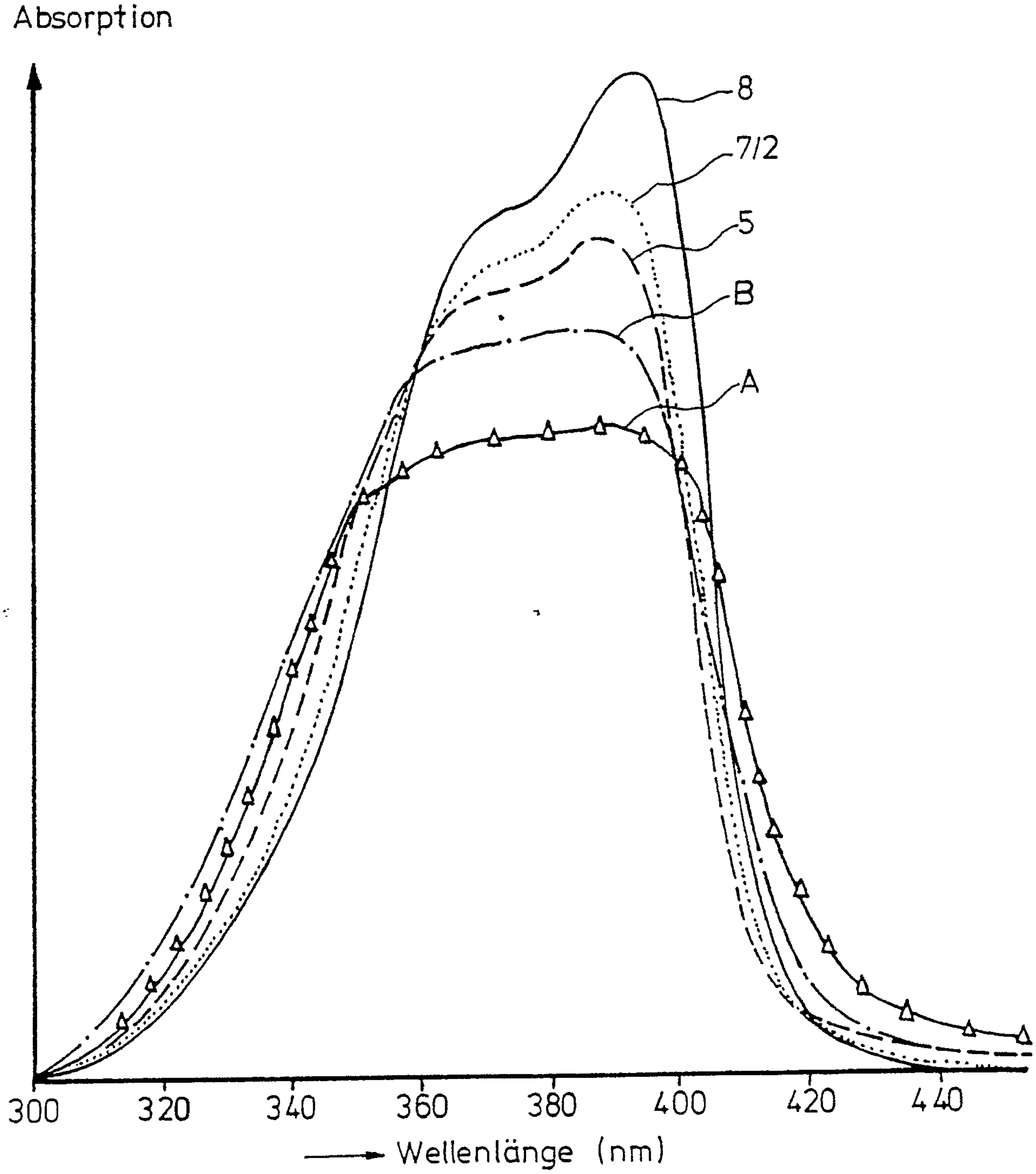
Absorption
8
7/2
5
B
A
300
320
340
360
380
400
420
440
Wellenlänge (nm)

FIG. 1

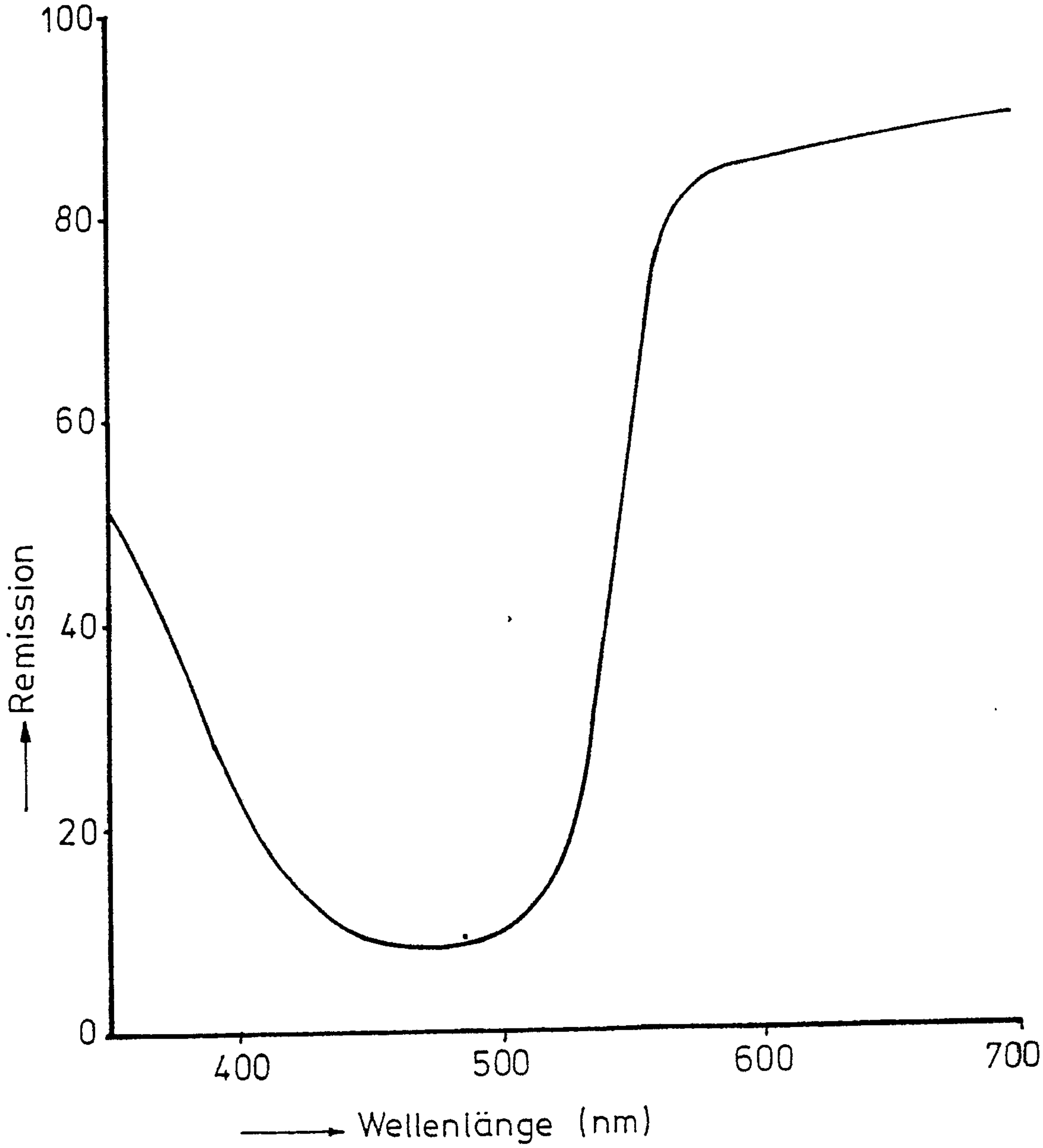


FIG. 2